# EUROPEAN PATENT SPECIFICATION

(11) **EP 1 714 544 B1**
(45) Date of publication and mention of the grant of the patent: **11.05.2011**
(21) Application number: 05710009.1
(22) Date of filing: 09.02.2005
(51) Int. Cl.: A01G 1/00, A01H 11/00, A01G 31/00

(54) **METHOD OF PRODUCING YOUNG MOSS SEEDLINGS, METHOD OF PRODUCING MOSS MAT.**
VERFAHREN ZUR HERSTELLUNG VON JUNGEN MOOSKEIMLINGEN, VERFAHREN ZUR HERSTELLUNG EINER MOOSMATTE.
PROCEDE DE PRODUCTION DE JEUNES PLANTS DE MOUSSE, PROCEDE DE PRODUCTION MATTE DE MOUSSE.

(30) Priority: 10.02.2004 JP 2004033525; 26.07.2004 JP 2004217231
(43) Date of publication of application: 25.10.2006
(73) Proprietor: Hero Corporation, Izumi-shi Osaka 594-1136 (JP); Murase, Harukito, Sakai-shi Osaka 590-0137 (JP); Inoue, Kouichi, Osaka-shi, Osaka 5340024 (JP)
(72) Inventor: Murase, Haruhiko, Sakai-shi, Osaka 590-0137 (JP); Inoue, Kouichi Liberty Stage 403, Osaka-shi Osaka 5340024 (JP)
(74) Representative: Scott, Susan Margaret
(86) International application number: PCT/JP2005/001957
(87) International publication number: WO 2005/074390

(56) References cited:
- EP-A1- 0 475 489
- WO-A-01/89291
- GB-A- 2 282 822
- JP-A- 8 228 620
- JP-A- 2000 510 325
- JP-A- 2003 269 748
- OKAYAMA T. ET AL: 'Mist Kansui o Mochiita Koke no Sokusei Saibai.' JAPANESE SOCIETY OF AGRICULTURAL MACHINERY. 01 September 2002, pages 429 - 430, XP002994268

## Description

### Field of the Invention

The present invention relates to a method of producing young moss seedlings, in particular, for young moss seedlings preferable as greenery plants, by which a plenty of young moss seedlings can be produced with high speed, and to a method of producing a moss mat in which moss is prepared in a mat-like state for convenient construction.

### Background of the invention

Usually, moss is cultivated under artificially natural atmosphere as a main material for gardening. This can be done because demand for moss is relatively little and therefore, cultivation under natural atmosphere can fully meet the demand. Moss is grown taking a long time of 2 to 3 years since the growth thereof is slow under the present technology.

Since moss prefers particular growth atmosphere, it is grown in few utilizable places as cultivation lands. Therefore, conventionally, some part of moss is shipped as landscape live moss and other part thereof is shipped as cut and dried moss species during production processes.

On the other hand, as a greenery plant, construction is being carried out with moss mats in which moss is prepared in mat-like covering and fixing on roofs of buildings, wall surfaces, road side walls, shore protection surfaces, and the like. Moss is resistant to climate changes as a greenery plant and grows without soil, and therefore, said condition is preferable since moss is light weighted and requires substantially no maintenance. A moss mat is a mat-like material, where moss grows in a cluster like a so-called grassy mat, and usually from the view point of handling moss for construction purposes and the like, a moss mat is defined as a moss got tangled in a substantially 40 to 60 cm-square on which buds of moss (ear-like ones) grow out on the upper surface of the mat. In general, a moss mat is produced by putting young moss seedlings in a base plate composed of a net with resin fibers knitted, one whose enveloping surface is plate-like with filament tangled, a resin-made frame by injection molding and the like, or combinations thereof, followed by a process called a rearing process in which moss is proliferated and length of moss is made to grow.

In order to produce moss mats, a large amount of moss is required, however, the actual condition is that production technology of moss has not yet been established. In particular, Racomitrium canescens which has strong resistance against dryness and which grows even on inorganic plates such as wall surfaces of buildings is preferable for a greenery plant in this regard, however, under natural environment, it grows very slow, therefore, it has been difficult to utilize as a greenery plant which requires mass cultivation.

Conventionally, technology has been proposed which uses moss as a greenery plant (Patent document 1). In said patent document 1, a method for repeatedly subculturing a gametophyte of moss indigene native to fields in a multidimensional manner by obtaining the gametophyte or a method for cultivating cells of moss plants by using culture medium has been proposed. (Patent document: page 4, column 7, line 29 to page 4, column 8, line 20).

Patent document 1: Patent No. 2863987

However, as these technologies take time and trouble, such technologies that produce a large amount of moss with high speed under artificially controlled environment have not always been provided. Further, even when young moss seedlings come out, in order to prepare moss mats convenient for construction, rearing of the young moss seedlings is necessary, that is, to let youngmoss seedlings proliferate inamossmat support medium, and allow rooting to grow in the direction of the half side of a mat. A mat body is reared under natural environment or under controlled environment with roofs and the like with water sprinkled to expedite moss growth. Since it takes several years or months for this rearing, it has also been a bottleneck on utilizing moss for greening.

The object of the present invention is to provide a method for producing a large amount of young moss seedlings under artificially controlled environment with high speed, and in particular, a method for producing young moss seedlings of Racomitrium canescens and the like suitable for greenery plants, and a method for producing moss mats in a short time.

GB 2, 282 822 discloses the production of moss seedlings by sterile culture of plant tissue. EP 475,489 discloses a method for producing ground cover provided with vegetation such as moss.

### SUMMARY OF THE INVENTION

As a result of intensive studies for solving the above problems, the inventors have found the unexpected phenomena that a large amount of regeneration buds (lateral buds) breed with high speed around gametophytes of moss which have initially been considered to wither away and die down when young moss seedlings are grown in nutrient solution without finding for growing environment of moss in natural environment, thereby completing the present invention based on this finding.

The present invention relates to a method of producing young moss seedlings according to claim 1. In this method, young moss seedlings are grown in nutrient solution. Moss mats may also be produced in nutrient solution, by the method of claim 12. In other words, the present invention relates to a method of producing moss controlling the growth of moss in nutrient solution.

In particular, young moss seedlings are preferably grown in nutrient solution within the range of a temperature of 0 to 60°C and of a photosynthetic active photon flux density of not greater than 200 (µmolm⁻²s⁻¹).

Gametophytes of moss are grown letting gaseous body including oxygen (e.g. air and the like) bubble in the nutrient solution. This produces intermittent contact with said gametophytes in nutrient solution by, for example, aerating and stirring. When young moss seedlings are grown in nutrient solution in this way, impact of light and gravity can be freely imparted from the 360-degree direction without limited to a certain direction, and regeneration buds can be bred around gametophytes, thereby capable of obtaining young moss seedlings preferable for greening. By this, for example, young moss seedlings in which enveloping surfaces at the tip parts of said bred regeneration buds are spindle-shaped preferable for greening can be produced.

In addition, young moss seedlings can also be grown by fixing said young moss seedlings in a certain direction in nutrient solution and also, young moss seedlings having regeneration buds with breeding directionality around gametophytes can be cultivated. Thus, since young moss seedlings are grown in nutrient solution, the positions of young moss seedlings in nutrient solution can be controlled and therefore, breeding forms suitable for various uses such as greenery uses can be imparted to young moss seedlings.

In addition, it is desirable that young moss seedlings are grown by repeating light periods and dark periods in cycles of 24 hours or less duration and that they are grown under low concentration of fertilizers of, for example, 0 to 1.0 in electric conductivity (mS/cm) in nutrient solution.

Further, like the above, young moss seedlings can be grown in nutrient solution in a state where the above mentioned young moss seedlings or gametophytes of moss are retained on a mat support mediumhaving an aperture portion at least on one surface, and further, by growing moss buds which outgrow from the aperture portion of a mat support medium in nutrient solution in the normal line direction, rearing of a moss mat can be conducted in a short time.

### BRIEF EXPLANATION OF THE DRAWINGS

Fig.1 is a view showing an image with 0 day passing from the onset of moss piece cultivation.
Fig.2 is a view showing Fig.1 in the form of a diagram.
Fig.3 is a view showing an image with 7 days passing from the same cultivation.
Fig.4 is a view showing Fig.3 in the form of a diagram.
Fig.5 is a view showing an image with 21 days passing from the same cultivation.
Fig.6 is a view showing Fig.5 in the form of a diagram.
Fig.7 is a view showing an image with 50 days passing from the same cultivation.
Fig.8 is a view showing Fig.7 in the form of a diagram.

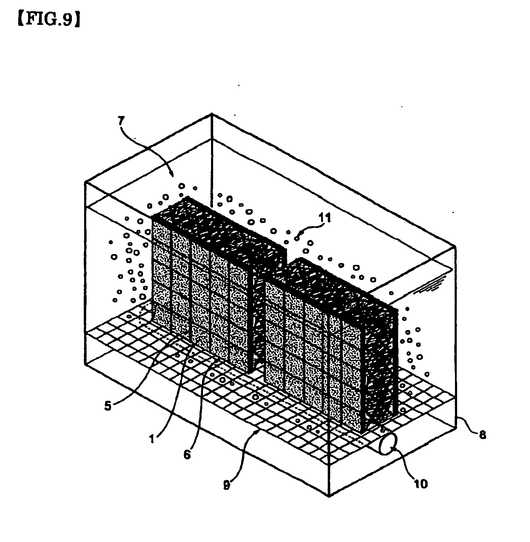

Fig.9 is a view showing a frame format of a cultivation state of Example 2.

### DETAILED DESCRIPTION OF THE EMBODIMENTS

Although moss used in the present invention is not specifically limited and regardless of mycorrhiza or sterile, any moss may be used so long as its regeneration bud breeds around a gametophyte grown in nutrient solution. For example, Bryopsida and Hepaticopsida may be used and in particular, a moss plant of Bryopsida is preferable. For example, in Bryopsida, Rhacomitrium Bird. including Racomitrium canescens, Racomitrium ericoides, Racomitrium japonicum, Racomitrium lanuginosum, Racomitrium heterostichum, Racomitrium laetum, Rhacomitrium sudeticum, Racomistrium fasciculare, Rhacomitrium anomodontoides, Racomistrium fasciculare, Rhacomitrium carinatum, Rhacomitrium aciculare, and the like, Thuidium B.S.G including Thuidium kanedae, Thuidium cymbifolium, Thuidium tamariscinum, Thuidium delicatulum, Thuidium recognitum, Thuidium pristocalix, Thuidium sparsifolium, and the like, Climacium Web et Mohr including Climacium japonicum, Climacium dendroides, and the like, Dicranum Hedw. including Dicranum scoparium, Dicranum japonicum, Dicranum nipponense, Dicranum fuscescens, Dicranum majus, Dicranum caesium, Dicranum polysetum, Dicranum drummondii, Dicranum flagellare, Dicranum mayrii, Dicranum viride, Dicranum fulvum, Dicranum hamulosum, Dicranum leiodontum, and the like, Hypnum Hedw. including Hypnum plumaeforme, Hypnum sakuraii, Hypnum oldhamii, Hypnum calcicolum, Hypnum fujiyamae, Hypnum cupressiforme, Hypnum tristo-viride, Hypnum pallescens, Hypnum reptile, Hypnum plicatulum, Hypnum subimponens, Hypnum erectiusculum, Hypnum lindbergii, and the like, Rhizogonium Brid. including Rhizogonium dozyanum, Rhizogonium spiniforme, Rhizogonium spiniforme, and the like can be used.

In Hepaticopsida, moss which belongs to Heteroscyphus Schiffn. including Heteroschyphus planus, Heteroscyphus argutus, Heteroschyphus bescherellei, Heteroscyphus lophocoleoides, Heteroscyphus tener, Chiloscyphus aselliformis, and the like, Porella. L including Porella perrottetiana, Porella stephaniana, Porella campylophylla, Porella caespitans,Porella japonica, Porella oblongifolia, Porella grandiloba, Porella vernicosa, Porella fauriei, Porella gracillima, and the like, Bazzania S. Gray including Bazzania japonica,Bazzania yoshinagana, Bazzania fauriana, Bazzania trilobata,Bazzania denuadata, Bazzania bidentula, Bazzania tricrenata, Bazzania pearsonii,Lepidozia pompeana, Bazzania tridens, Bazzania mayebarae, and the like can be used.

In particular, Rhacomitrium Canescens including Racomitrium canescens, Racomitrium ericoides, Racomitrium japonicum, and the like are preferable for greening of buildings. This moss requires no soil or fertilizer for growth and can survive in very dried condition without putting excess burden on wall surfaces of buildings, for example. Although growth speed of this kind of a young seedling is very slow under natural environment, in the present invention, it can be cultivated in mass with high speed.

Material moss (material seedling) grown in nutrient solution can be obtained by collecting gametophytes, in particular, gametophytes having forms of leafy gametophytes from moss indigene native to fields or commercially available one.

Temperature of nutrient solution is 0 to 60°C, preferably 5 to 50°C, and further preferably 15 to 25°C. When temperature of nutrient solution is less than 0°C, it is not preferable since the nutrient solution freezes. When temperature of nutrient solution exceeds 60°C, microbes and the like is likely to breed, which slows moss growth, and therefore, it is not preferable, either.

Concentration of a fertilizer of nutrient solution is 0 to 1.0 in electric conductivity (mS/cm) and preferably 0 to 0.2, and nutrient solution preferably includes a nutrient solution with low concentration or no fertilizer included. As a fertilizer, a general fertilizer (for example, HYPONeX®) can be used. When concentration of a fertilizer exceeds 1. 0 (mS/cm), since it is too thick as one, growth of a young moss seedling is likely to slow and therefore, it is not preferable.

Nutrient solution preferably includes phytohormone. As phytohormone, ethylene, abscisic acid, auxin (indoleacetic acid), cytokinin (zeatin), gibberellin (gibberellic acid), and the like can be exemplified, however, plant growth hormone is preferable and among them, auxin, cytokinin, and gibberellin are preferable. Gibberellin is the most preferable for large growth promoting effect. They may be used alone, may be used in mixtures and/or may be used together. When concentration of plant growth hormone is high, it causes physiological disorder of a plant, therefore, concentration of 0.01 to 0.2 ppm is preferable, diluted with about 1000 times compared with concentration used for general gardening, and concentration of 0.1 ppm is further preferable.

Light is required for growth of moss. Light may be natural or artificial and light source is not specially limited, however, it is necessary that the light has ingredients of wavelength regions which enables photosynthesis and which do not inhibit growth. When growing moss under artificial environment, a general incandescent lamp, fluorescent lamp, mercury lamp, LED, and the like can be used, however, LED is preferable from the view point of low energy cost, easy heat control, and easy maintenance, and among them, LEDwhich emits red light, or orange light is preferable. When LED is incorporated in a moss cultivating device, use of LED arranged in the form of a two-dimensional array will make it compact. Therefore, it becomes easy to prepare a cultivation container which has a flat bubbler connected to an air pipe in which nutrient solution and gametophytes of moss are put and an LED array as one unit, and said unit is piled up three-dimensionally in multiples. An LED array is preferable for a long life due to low electric energy and little waste heat.

Photosynthetic active photon flux density(PPFD)is preferably not greater than 200 (µmolm⁻²s⁻¹), particularly preferably not greater than 50 (µmolm⁻²s⁻¹), and further preferably 20 to 30 (µmolm⁻²s⁻¹) which is not less than light compensation point. When photosynthetic active photon flux density is high, it is not preferable since growth of young moss seedlings is likely to slow. For information, when young moss seedlings are grown in nutrient solution, it is preferable to dim a light by repeating light and dark periods. Inparticular, it is preferable that youngmoss seedlings are grown by repeating light periods and dark periods in cycles of 24 hours or less duration. By repeating light periods and dark periods at specified cycles, the balance between photosynthesis and metabolism are secured and therefore, it is preferable. Therefore, for example, by setting the cycle time every 12 hour initially and then every 6 hour after a passage of a specified time, for example, the cycle of light periods and dark periods depending on the growth stages of young seedlings is controlled, thereby capable of controlling breeding amount and speed of regeneration buds.

The direction of regeneration buds generated by light stimulation is affected by gravitation, light direction, oxygen concentration, and the like. The growing direction of lateral buds has positive photoaxis. When light is emitted to moss gametophytes suspended in nutrient solution from 360° direction, regeneration buds are generated in every direction. For this reason, young moss seedlings in which an enveloping surface at the tip of said bred regeneration buds are spindle-shaped are generated. On the other hand, since growing direction of regeneration buds can be controlled by the light direction, when the light is emitted from the direction where lateral buds are desirably grown in designing moss mats, regeneration buds grow in the incidence direction of the light.

When gametophytes of moss are filled in a moss mat support medium and are grown in nutrient solution in a state where free motion of gametophytes is prohibited, light is emitted from the vertical direction to an aperture portion of a moss mat support medium and regeneration buds are generated toward the aperture portion. And by taking said regeneration buds as they are out of nutrient solution, further followed by rearing them with light emitted from the vertical direction to an aperture portion of a moss mat support medium, a moss mat can be produced in which so-called moss ears are grown vertically to its surface from a mat aperture portion.

On the other hand, when gametophytes of moss are filled in a moss mat support medium and are grown in nutrient solution in a state where free motion of gametophytes is prohibited, light is emitted from the vertical direction to an aperture portion of a moss mat medium and regeneration buds are generated toward the aperture portion. And by keeping on cultivating in nutrient solution, a moss mat can be produced at once in which tips of regeneration buds are grown vertically to its surface from a mat aperture portion like a so-called moss ears.

In addition, in the method of the present invention, it is important to let a gaseous body which includes oxygen contact with gametophytes of moss in nutrient solution intermittently causing aerating and stirring, thereby making them grow.

By this, regeneration buds which used to wither away or die down in liquid breed and proliferate.

For information, devices for carrying out the method of the present invention, for adjusting environment at a constant temperature and constant light, a complete control type is preferable and by the complete control type, previous step of plant factories preferable for mass production can be made up. Therefore, when young seedlings of moss obtained by the above method are transferred to a medium for a moss mat under a plant factory environment and reared, a large amount of regeneration buds bred and proliferated from a large amount of gametophytes are further grown under controlled environment of a plant factory, in particular, under computer control, and therefore, a large amount of moss community can be produced, which can formulate a system as a production factory of a series of greenery moss plants.

### EXAMPLES

### Example 1

A sample was prepared using a moss piece (15mm) obtained by cutting a part of wild Racomitrium canescens (gametophytes with leafy gametophytes) obtained from mountain fields in Izumi-shi, Osaka-fu, and 25 g of the sample was put in a 500 ml culture tank, followed by stirring by bubbling air under completely controlled environment with 50 (µmolm⁻²s⁻¹) of photosyntheticactive photon flux density (PPFD) by a fluorescent light, 12 hours of light and dark periods, 15°C of nutrient solution temperature, and HYPONeX® with fertilization concentration of 0.2 (mS/cm), thereby cultivating young seedlings.

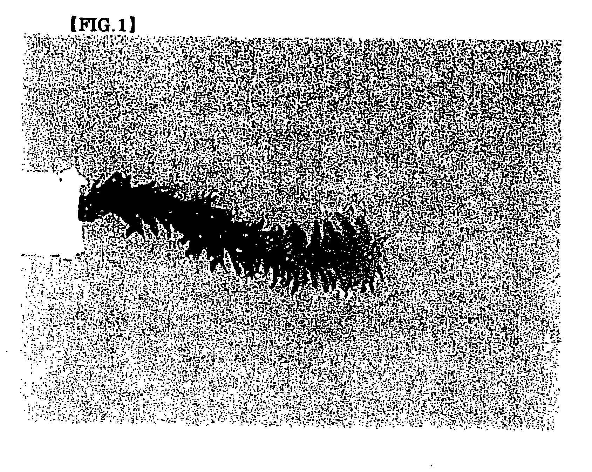

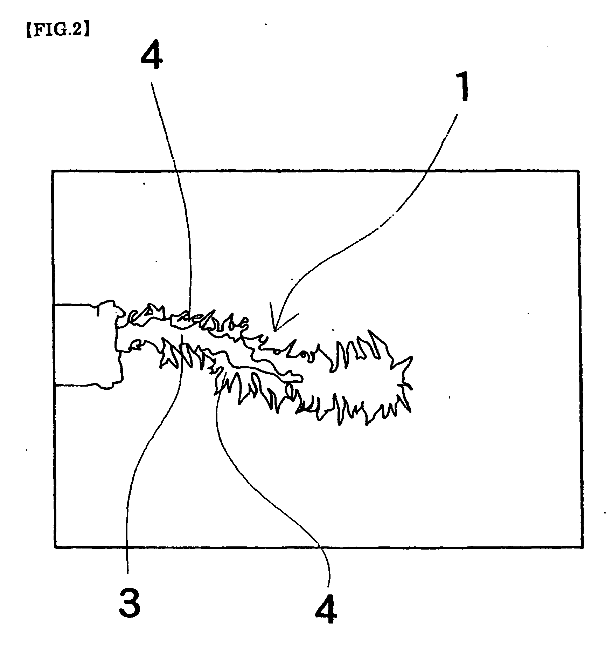

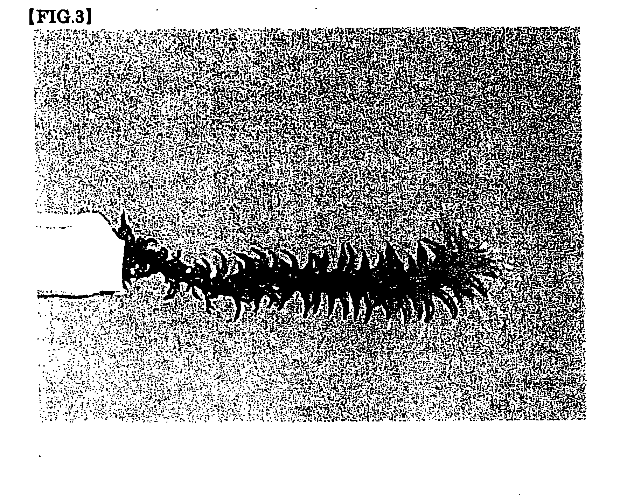

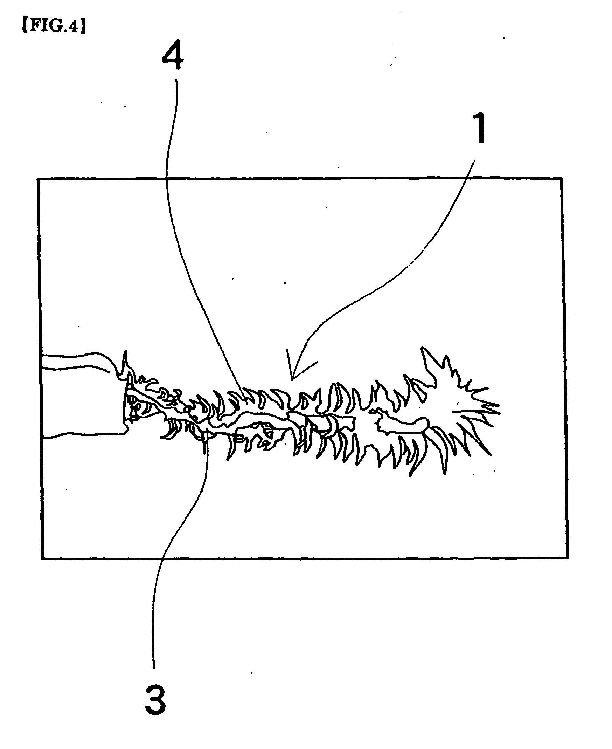

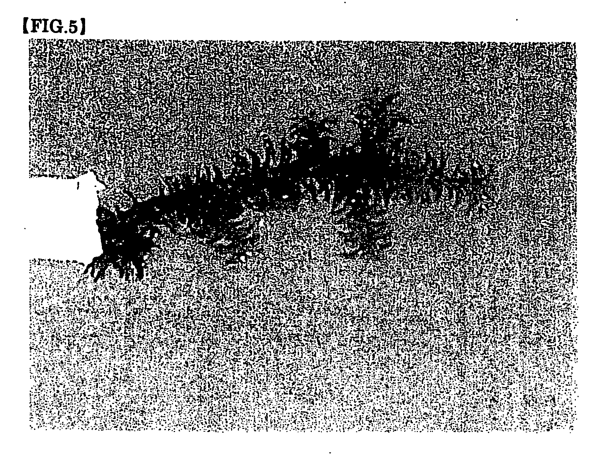

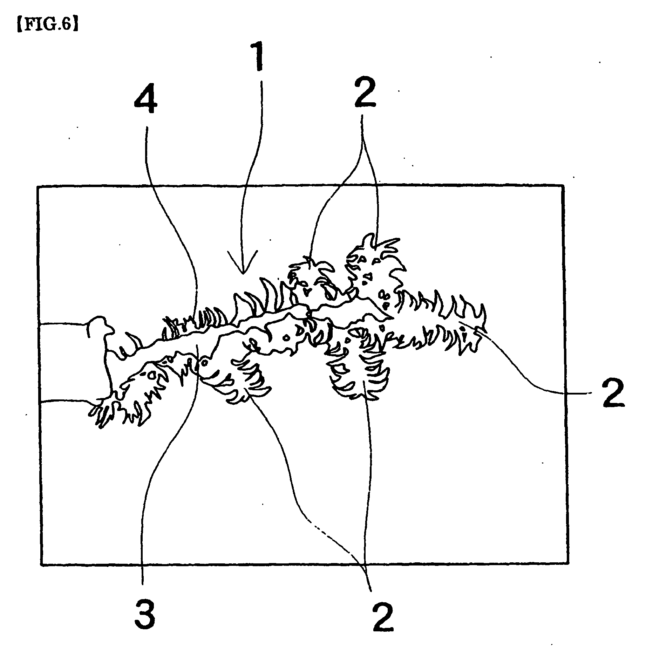

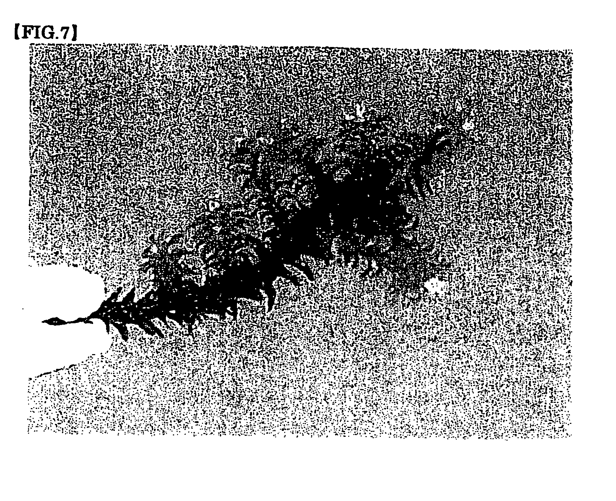

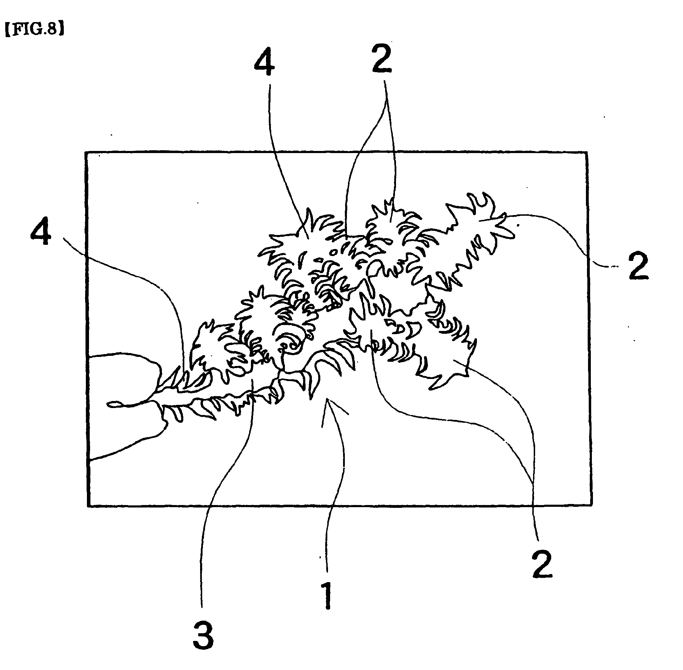

Fig.1 is a view showing an image with 0 day passing from the onset of moss piece cultivation, Fig.2 is a view showing Fig. 1 in the form of a diagram, Fig. 3 is a view showing an image with 7 days passing from the same cultivation, Fig.4 is a view showing Fig.3 in the form of a diagram, Fig.5 is a view showing an image with 21 days passing from the same cultivation, Fig.6 is a view showing Fig.5 in the form of a diagram, Fig.7 is a view showing an image with 50 days passing from the same cultivation, and Fig.8 is a view showing Fig.7 in the form of a diagram. Every image diagram shows moss pieces enlarged by about three times image-processed on a computer after digitally photographing the moss pieces.

As shown in Fig.1 (Fig.2), in gametophyte 1 with 0 day passing from the onset of cultivation, only leafy gametophyte 4 is around a leaf stalk 3, while on the other hand, as shown in Fig.5 (Fig.6) and Fig.7 (Fig.8), from 21 days to 50^{h} days passing from the onset of cultivation, plural of or multiple of regeneration buds 2 clearly breed and proliferate around gametophyte 1. It is considered that regeneration buds 2 breed from a withered or dormant leaf stalk 3. By this, according to such a method, young moss seedlings suitable for greenery plants in which regeneration buds 2 breed all around the moss gametophyte 1.

Under the above mentioned environmental condition, the growth speed of each young moss seedling was about 5 mm in 3 weeks. This is about 8 times as fast as the growth speed under natural environment.

In addition, in the case young moss seedlings are produced as a front-end of the plant factory environment, and when a production method as mentioned above is employed, for example, since the design can be employed in proportion as the capacity of a culture tank with regard to culture amount, young seedlings equivalent to 0.25m² can be grown in a 5 litter tank.

### Example 2

A casing 6 having a parallelepiped stereoscopic structure was formed by filling moss pieces 1 (average length of 15 mm) as used in Example 1 densely in a polyolefin stereoscopic net 5 for a moss mat of 5cm in length, 5cm in width, and 1.5 cm in thickness which is a moss mat support medium stereoscopically woven with polyethylene fibers, thereby restricting the mutual motion of moss pieces. Two casings in total together with other casing 6 manufactured by the same method were fixed on an air-permeable partition wall 9 in a culture container 8 by dipping the lateral surface of the above mentioned casings in a vertical direction in a transparent culture container 8 filled with nutrition solution 7 of HYPONeX® diluted by 1000 times.

Cultivation was continued, pumping air from an air supply tube 10, letting air bubble 11 generate so that enough amount of air can be supplied from a flat-type bubbler (trade name "AIRSTONE" not illustrated) attached to the air supply tube 10 to the casing 6, stirring nutrient solution by bubbling, irradiating light so that an irradiated surface on which moss pieces are filled in polyolefin stereoscopic net 5 intermittently every 12 hour from the front face of a cultivation container has PPFD of 50µmolm⁻²s⁻¹ and maintaining liquid temperature at 15 °C. Moss growth was substantially the same as in Example 1 in which moss alone was subject to hydroponic cultivation.

### Example 3

One of the casings of Example 2 was taken out from the cultivation container 3 weeks after the onset of cultivation and was put on a concrete, sprinkling water from time to time as in ordinary rearing, rearing to be exposed to sunlight above thereby obtaining a moss mat.

### Example 4

Further cultivation was continued for 5 weeks regarding the other remaining casing. A moss mat was produced in which the tip of moss regeneration buds began to grow by 5 mm from an aperture portion on a light irradiated net surface.

### Example 5

Young moss seedlings were cultivated as in Example 1 except that gibberellin was added with the concentration of 0.1 ppm in nutrient solution. The growth speed of young moss seedlings was about 5 mm in 1 week, respectively.

### Effect of the invention

By this, when gametophytes are obtained from moss indigene native to fields and the gametophytes are put in a tank in which nutrient solution is stored, for example, regeneration buds are bred and proliferate around the gametophytes of moss with high speed, and therefore, a large amount of moss can be produced with high speed. And by growing young moss seedlings under controlled environment, moss community suitable for greening can be provided.

Further, by rearing moss mat bodies in nutrient solution, moss mats convenient for construction can easily be produced in a short time. Young moss seedlings with peculiar forms in which regeneration buds proliferate around the gametophytes of moss are obtained.

### Industrial Applicability

According to the method of the present invention, since a large amount of young moss seedlings can be produced with high speed under artificially controlled environment, plant factory system for growing moss including young seedling cultivation facilities can be built up. Further, since a large amount of moss mats can be produced with high speed, moss plants can be put to practical uses for the first time as greenery plants in construction fields or environment-related fields including greening of roofs, wall surfaces, and the like. In addition, young moss seedlings obtained by the present production method can be applied to various purposes such as gardening, landscaping, medical purposes and the like not only to the purpose of greenery plants.

## Claims

1. A method for producing young moss seedlings wherein gametophytes having leafy gametophytes of moss are grown in nutrient solution which is stirred by bubbling with a gaseous body including oxygen.

2. The method of production set forth in claim 1, wherein the gaseous body is air.

3. The method of production as set forth in claim 1 or claim 2 which comprises growing the gametophytes within the range of a temperature of 0 to 60°C, and photosynthetic active photon flux density(PPFD)of not greater than 200 (µmolm⁻²s⁻¹) respectively.

4. The method of producing young moss seedlings as set forth in any one of claims 1 to 3, wherein moss is grown by repeating light periods and dark periods in cycles of 24 hours or less duration.

5. The method of producing young moss seedlings as set forth in any one of claims 1 to 4, wherein fertilizer concentration of said nutrient solution is 0 to 1.0 (mS/cm).

6. The method of producing young moss seedlings as set forth in any one of claims 1 to 5, wherein said nutrient solution includes phytohormone.

7. The method of producing young moss seedlings as set forth in claim 6, wherein said phytohormone includes at least one from gibberellin, cytokinin, and auxin.

8. The method of producing young moss seedlings as set forth in any one of claims 1 to 7, wherein said young seedlings are moss plants of Bryopsida.

9. The method of producing young moss seedlings as set forth in claim 8, wherein said moss plants are Racomitrium canescens.

10. The method of producing young moss seedlings as set forth in any one of claims 1 to 9, wherein said young moss seedlings are used as moss for greening.

11. The method of producing a moss plant for greening, wherein young moss seedlings obtained by the method of production set forth in any one of claims 1 to 9 are placed on a moss mat support medium under plant factory atmosphere and reared.

12. The method of producing a moss mat, wherein moss is reared in nutrient solution which is stirred by bubbling with a gaseous body including oxygen in a state where gametophytes having leafy gametophytes of moss are retained in said mat support medium with an aperture portion at least on one surface.

13. The method of producing a moss mat as set forth in claim 12, wherein said nutrient solution is irradiated with light from the normal line direction of a mat surface.

## Patentansprüche

1. Verfahren zur Herstellung junger Mooskeimlinge, wobei Gametophyten, die begrünte Moosgametophyten aufweisen, in Nährlösung wachsen gelassen werden, welche durch Blubbern mit einem gasförmigen Körper, der Sauerstoff enthält, gerührt wird.

2. Das in Anspruch 1 dargelegte Verfahren zur Herstellung, wobei der gasförmige Körper Luft ist.

3. Das Verfahren zur Herstellung wie in Anspruch 1 oder Anspruch 2 dargelegt, welches das Wachsen der Gametophyten innerhalb eines Temperaturbereichs von 0 bis 60 °C beziehungsweise eine photosynthetisch aktive Photonenflussdichte (PPFD) von nicht mehr als 200 (µmolm⁻²s⁻¹) umfasst.

4. Das Verfahren zur Herstellung junger Mooskeimlinge wie in einem der Ansprüche 1 bis 3 dargelegt, wobei Moos durch sich in Zyklen von 24 Stunden oder kürzerer Dauer wiederholenden Zeitintervallen mit Beleuchtung und Zeitintervallen mit Dunkelheit wachsen gelassen wird.

5. Das Verfahren zur Herstellung junger Mooskeimlinge wie in einem der Ansprüche 1 bis 4 dargelegt, wobei die Düngerkonzentration der Nährlösung 0 bis 1,0 (mS/cm) ist.

6. Das Verfahren zur Herstellung junger Mooskeimlinge wie in einem der Ansprüche 1 bis 5 dargelegt, wobei die Nährlösung Phytohormon enthält.

7. Das Verfahren zur Herstellung junger Mooskeimlinge wie in Anspruch 6 dargelegt, wobei das Phytohormon mindestens eins aus Gibberellin, Cytokinin und Auxin enthält.

8. Das Verfahren zur Herstellung junger Mooskeimlinge wie in einem der Ansprüche 1 bis 7 dargelegt, wobei die jungen Keimlinge Moospflanzen von Bryopsida sind.

9. Das Verfahren zur Herstellung junger Mooskeimlinge wie in Anspruch 8 dargelegt, wobei die Moospflanzen Graue Zackenmützen (Racomitrium canescens) sind.

10. Das Verfahren zur Herstellung junger Mooskeimlinge wie in einem der Ansprüche 1 bis 9 dargelegt, wobei die jungen Mooskeimlinge als Moos zur Begrünung verwendet werden.

11. Verfahren zur Herstellung einer Moospflanze zur Begrünung, wobei die jungen Mooskeimlinge, erhalten durch das in einem der Ansprüche 1 bis 9 dargelegte Verfahren zur Herstellung, auf einem Moosmatten-Trägermedium unter Gewächshaus-Atmosphäre platziert werden und aufgezogen werden.

12. Verfahren zur Herstellung einer Moosmatte, wobei Moos in Nährlösung, welche durch Blubbern mit einem gasförmigen Körper, der Sauerstoff enthält, gerührt wird, in einem Zustand aufgezogen wird, in welchem Gametophyten, die begrünte Moosgametophyten aufweisen, in dem Mattenträgermedium mit einem Öffnungsbereich an mindestens einer Oberfläche gehalten werden.

13. Das Verfahren zur Herstellung einer Moosmatte wie in Anspruch 12 dargelegt, wobei die Nährlösung mit Licht aus Richtung der Normallinie einer Mattenoberfläche bestrahlt wird.

## Revendications

1. Procédé pour produire de jeunes semis de mousse dans lequel des gamétophytes ayant des gamétophytes feuillus de mousse sont mis à croître dans une solution nutritive qui est agitée par barbotage d'un corps gazeux contenant de l'oxygène.

2. Procédé de production selon la revendication 1, dans lequel le corps gazeux est l'air.

3. Procédé de production selon la revendication 1 ou la revendication 2, qui comprend la croissance des gamétophytes à une température située dans la gamme allant de 0 à 60°C avec une densité de flux de photons photosynthétiquement actifs (PPFD) non supérieure à 200 (µmol m⁻²s⁻¹), respectivement.

4. Procédé pour produire de jeunes semis de mousse selon l'une quelconque des revendications 1 à 3, dans lequel la mousse croît au moyen d'une répétition de périodes de lumière et de périodes d'obscurité en cycles ayant une durée de 24 heures ou moins.

5. Procédé pour produire de jeunes semis de mousse selon l'une quelconque des revendications 1 à 4, dans lequel la concentration de fertilisant de ladite solution nutritive est de 0 à 1,0 (mS/cm).

6. Procédé pour produire de jeunes semis de mousse selon l'une quelconque des revendications 1 à 5, dans lequel ladite solution nutritive contient une phythormone.

7. Procédé pour produire de jeunes semis de mousse selon la revendication 6, dans lequel ladite phythormone comprend au moins l'une parmi la gibbérelline, la cytokinine, et l'auxine.

8. Procédé pour produire de jeunes semis de mousse selon l'une quelconque des revendications 1 à 7, dans lequel lesdits jeunes semis sont des plants de mousse Bryopsida.

9. Procédé pour produire de jeunes semis de mousse selon la revendication 8, dans lequel lesdits plants de mousse sont Racomitrium canescens.

10. Procédé pour produire de jeunes semis de mousse selon l'une quelconque des revendications 1 à 9, dans lequel lesdits jeunes semis de mousse sont utilisés en tant que mousse pour écologisation.

11. Procédé pour produire un plant de mousse pour écologisation, dans lequel de jeunes semis de mousse obtenus par le procédé de production de l'une quelconque des revendications 1 à 9 sont placés sur un milieu de support de tapis de mousse dans l'atmosphère d'une manufacture de plantes et cultivés.

12. Procédé pour produire un tapis de mousse, dans lequel une mousse est cultivée dans une solution nutritive qui est agitée par barbotage d'un corps gazeux contenant de l'oxygène dans un état où des gamétophytes ayant des gamétophytes feuillus de mousse sont retenus dans ledit milieu de support de tapis ayant une partie d'ouverture sur au moins une surface.

13. Procédé pour produire un tapis de mousse selon la revendication 12, dans lequel ladite solution nutritive est irradiée avec une lumière à partir de la direction linéaire normale d'une surface de tapis.
